# EUROPÄISCHE PATENTSCHRIFT

(11) **EP 3 106 193 B1**
(45) Veröffentlichungstag und Bekanntmachung des Hinweises auf die Patenterteilung: **26.12.2018**
(21) Anmeldenummer: 15172153.7
(22) Anmeldetag: 15.06.2015
(51) Int. Cl.: A61M 5/32

(54) **SPRITZENKÖRPER**
SYRINGE BODY
SERINGUE

(43) Veröffentlichungstag der Anmeldung: 21.12.2016
(73) Patentinhaber: Gerresheimer Regensburg GmbH, 93047 Regensburg (DE); Gerresheimer Bünde GmbH, 32257 Bünde (DE)
(72) Erfinder: Wittland, Frank, 32257 Bünde (DE); Vogl, Maximilian, 92708 Mantel (DE)
(74) Vertreter: Hannke, Christian

(56) Entgegenhaltungen:
- FR-A1- 2 884 723
- US-A- 2 834 346
- US-A- 4 932 940
- US-A- 4 966 592
- US-A- 5 591 138
- US-A1- 2011 319 833

## Beschreibung

Die Erfindung betrifft einen Spritzenkörper mit einer endseitig angeordneten Sicherheitseinrichtung zur Vermeidung von Stichverletzungen, wobei der Spritzenkörper ein endseitig angeordnetes Stechelement umfasst und die Sicherheitseinrichtung mindestens einen Führungsstift und eine Ausnehmung und aufweist, mittels welcher eine Führungskulisse ausgebildet ist, um den Führungsstift bei einer Relativbewegung von Spritzenkörper zu Sicherheitseinrichtung in einer Längsrichtung des Spritzenkörpers zu führen.

Aus dem Stand der Technik sind bisher Sicherheitseinrichtungen zur Vermeidung von Stichverletzungen bekannt, die nach einem Befüllen der Spritzen um die gesamte Spritze herum montiert werden.

Diese Sicherheitsvorrichtungen vergrößern jedoch die Dimension, sowohl in der reinen Größe als auch dem Gewicht, der Spritze zum Gebrauch, so dass eine Handhabung von Spritzen mit derartigen Sicherheitsvorrichtungen erheblich erschwert wird.

Darüber hinaus sind Sicherheitseinrichtung derart konstruiert, dass eine Spritze bereits unbrauchbar gemacht werden kann, beispielsweise bei einem Abbruch der Injektion, ohne dass die Nadelspitze überhaupt aus der Sicherheitseinrichtung herausgetreten ist. Die Spritze ist also bereits unbrauchbar, bevor sie überhaupt Kontakt mit dem Patienten hatte und eigentlich noch gar nicht verunreinigt worden ist.

Andere bekannte Sicherheitseinrichtung geben den umgekehrten Fall wider. Die Nadelspitze wird erst dann in eine sichere Stellung überführt, wenn die Nadelspitze bereits aus der Sicherheitseinrichtung ausgetreten ist. Dies hat den gravierenden Nachteil, dass eine Injektion wiederholt werden könnte, obwohl die Nadel bereits mit dem Patienten in Kontakt war. Es ist daher möglich, dass jemand mit einer verunreinigten Spritze in Kontakt kommen kann und sich daran verletzen oder sogar infizieren kann. Gattungsgemäße Sicherheitsvorrichtungen, welche den Oberbegriff von Anspruch 1 offenbaren, sind aus US 2011/0319833, US4966592 und US5591138 bekannt.

Es ist daher die Aufgabe der vorliegenden Anmeldung, einen Spritzenkörper mit einer Sicherheitsvorrichtung bereitzustellen, die die Nachteile des Standes der Technik nicht mehr aufweist.

Gelöst wird diese zugrunde liegende Aufgabe von einem Spritzenkörper mit unter anderem einer endseitig angeordneten Sicherheitseinrichtung zur Vermeidung von Stichverletzungen, wobei der Spritzenkörper ein endseitig angeordnetes Stechelement umfasst und die Sicherheitseinrichtung zumindest eine Ausnehmung und mindestens einen Führungsstift aufweist, wobei mittels der Ausnehmung eine Führungskulisse ausgebildet ist, um den Führungsstift bei einer Relativbewegung von Spritzenkörper zu Sicherheitseinrichtung in einer Längsrichtung des Spritzenkörpers zu führen, wobei die Führungskulisse einen ersten und einen zweiten Kulissenbereich umfasst, die durch eine in einer Längsrichtung des Spritzenkörpers verlaufenden fiktiven Trennlinie voneinander getrennt sind und wobei der Führungsstift in einer Ausgangsstellung in dem ersten Kulissenbereich anordenbar ist und von dem ersten in den zweiten Kulissenbereich in eine Endstellung durch Überschreiten der Trennlinie überführbar ist, wenn ein distales Ende des Stechelements bei der Relativbewegung von Spritzenkörper zu Sicherheitseinrichtung auf der Höhe einer Austrittsöffnung der Sicherheitseinrichtung angeordnet ist.

Der Begriff "endseitig angeordnet" ist in diesem Zusammenhang derart zu verstehen, dass das jeweilige bauliche Element auf einem distalen Ende angeordnet ist. So sind die Sicherheitseinrichtung sowie das Stechelement an dem distalen Ende des Spritzenkörpers angeordnet.

Unter dem Begriff "Stechelement" ist eine Nadel, eine Kanüle, eine Lanzette oder dergleichen zu verstehen.

Unter einer "Ausgangstellung" wird eine Stellung, vorzugsweise des Führungsstiftes, beschrieben, die einer unbenutzten Spritze entspricht, also vor dem Gebrauch einer Spritze.

Unter einer "Endstellung" wird eine Stellung beschrieben, in der die vorliegende Spritze bereits benutzt wurde. Hat der Führungsstift die Endstellung erreicht, so ist es nicht mehr möglich, die Spritze noch einmal zu verwenden.

Erfindungsgemäß ist der Führungsstift von dem ersten Kulissenbereich in den zweiten Kulissenbereich überführbar. Diese Überführung findet statt, wenn der Führungsstift eine fiktive Trennlinie, die den ersten und den zweiten Kulissenbereich voneinander trennt, überschreitet. Befindet sich der Führungsstift in dem ersten Kulissenbereich, also in einer Ausgangsstellung, so wurde die Spritze noch nicht ausgelöst, d.h. die Nadel hat die Sicherheitseinrichtung noch nicht verlassen. Befindet sich der Führungsstift in dem zweiten Kulissenbereich, so ist die Nadel aus der Sicherheitseinrichtung bereits ausgetreten, so dass eine Injektion möglich ist. Beim Übergang von dem ersten Kulissenbereich zu dem zweiten Kulissenbereich, also genau dann, wenn der Führungsstift die Trennlinie überschreitet, befindet sich das distale Ende des Stechelements, beispielsweise der Nadel, auf der Höhe der Austrittsöffnung der Sicherheitseinrichtung.

Gemäß einer besonders bevorzugten Ausführungsform umfasst die Sicherheitseinrichtung mindestens ein Federelement. Das Federelement ist vorzugsweise mit dem Spritzenkörper wirkverbunden und wirkt der Relativbewegung des Spritzenköpers gegenüber der Sicherheitseinrichtung entgegen. Dies bedeutet, dass wenn ein Benutzer der Spritze den Spritzenkörper gegenüber der Sicherheitseinrichtung bewegt, so wirkt das Federelement entgegen und würde, falls die Relativbewegung unterbunden wird, den Spritzenkörper bezüglich der Sicherheitseinrichtung wieder in eine Position zurückführen, in der sich die Spritze wieder vollständig in der Sicherheitseinrichtung befindet.

Durch das Federelement kann also gewährleistet werden, dass die Nadel nach Gebrauch der Spritze sicher in die Sicherheitseinrichtung rückgeführt werden kann und der Führungsstift in die Endstellung vorzugsweise automatisch überführt werden kann.

Dabei kann das Federelement auf verschiedene Weisen ausgestaltet sein. Vorzugsweise handelt es sich bei dem Federelement um eine Spiralfeder.

Gemäß einer bevorzugten Ausführungsform ist die Sicherheitseinrichtung mittels eines Montageelements mit dem Spritzenkörper zumindest wirkverbunden. Es ist dabei denkbar, dass der Führungsstift/die Führungsstifte auf dem Montagelement angeordnet sind. Dabei ist vorteilhaft das Montageelement einerseits mit einem Nadelaufsatz, der mit dem Spritzenkörper verbunden ist, verbunden und andererseits mittels des zumindest einen Führungsstifts mit der Sicherheitseinrichtung verbunden, da der Führungsstift innerhalb der Führungskulisse der Sicherheitseinrichtung angeordnet ist.

Besonders vorteilhaft ist das Montageelement innerhalb einer Hülse der Sicherheitseinrichtung angeordnet. Weiter vorteilhaft weist eben diese Hülse auch die Führungskulisse auf. Gemäß einer bevorzugten Ausführungsform umfasst die Sicherheitseinrichtung, insbesondere die Hülse, zwei Ausnehmungen, und das Montageelement zwei Führungsstifte, wobei die Ausnehmungen und die Führungsstifte vorteilhaft gegenüberliegend ausgebildet sind, wodurch eine besonders vorteilhafte Führung gewährleistet werden kann.

Weiter bevorzugt ist das Federelement ebenso innerhalb der Hülse angeordnet und besonders bevorzugt mittels des Montageelements in der Hülse gegen ein Herausfallen gesichert.

Erfindungsgemäß weist der erste Kulissenbereich einen ersten kurvenförmigen Kulissenabschnitt und der zweite Kulissenbereich einen zweiten kurvenförmigen Kulissenabschnitt auf, an denen der Führungsstift, je nach Position in der Führungskulisse, geführt wird. Vorteilhaft kann der jeweilige Kulissenabschnitt mehrere Teilabschnitte aufweisen.

Da die Kulissenbereiche kurvenförmige Kulissenabschnitte aufweisen ist es vorteilhaft, wenn das Montageelement, wenn es auf dem Spritzenkörper montiert ist, axial nicht mehr verschoben kann, sich jedoch radial um den Spritzenkörper drehen kann. Dies gewährleistet, dass der Führungsstift dem Verlauf der jeweiligen Kulisse folgen kann. Dies bedeutet also, dass bei einer Relativbewegung das Montageelement und demzufolge auch der Führungsstift sich um den Spritzenkörper drehen können.

Erfindungsgemäß ist vorgesehen, dass an dem ersten sowie dem zweiten Kulissenbereich ein Trennbereich angrenzt. Dabei ist dieser Trennbereich zumindest teilweise zwischen dem ersten und dem zweiten Kulissenbereich anordenbar.

Erfindungsgemäß ist der Trennbereich derart ausgestaltet, dass der Trennbereich einen Scheitelpunkt aufweist, der auf der fiktiven Trennlinie. liegt. Vorteilhaft handelt es sich bei diesem Scheitelpunkt um ein lokales Extremum des Trennbereichs, so dass hierdurch eine Bewegung des Führungsstiftes unterstützt werden kann. Dadurch, dass der Scheitelpunkt auf der Trennlinie liegt, kann der erste als auch der zweite Kulissenbereich mittels des Trennbereichs und der Trennlinie gut bestimmt werden. Außerdem wird dadurch, dass es sich bei dem Scheitelpunkt vorzugsweise um ein lokales Extremum handelt, die Führung des Führungsstifts, insbesondere entgegen der Längsrichtung des Spritzenkörpers, geführt, da der Scheitelpunkt im Bereich um den Scheitelpunkt herum bestimmt, in welchen Kulissenbereich der Führungsstift geführt wird.

Gemäß einer besonders bevorzugten Ausführungsform ist, wenn der Führungsstift sich in dem zweiten Kulissenbereich befindet, der Führungsstift mittels einer Kulisse des zweiten Kulissenbereichs in einen Endbereich überführbar. Vorzugsweise ist in diesem Endbereich der Führungsstift mit einem Stoppelement wirkverbindbar.

Vorteilhaft handelt es sich bei der Kulisse des zweiten Kulissenbereichs um den zweiten Kulissenabschnitt.

Dies bedeutet also, wenn sich der Führungsstift im zweiten Kulissenbereich befindet, wird der Führungsstift mittels dem zweiten Kulissenbereichs und vorzugsweise mit Hilfe des Federelements in den Endbereich überführt und ist dann mit dem Stoppelement wirkverbunden.

Das Stoppelement ist dabei derart ausgestaltet, dass, wenn sich der Führungsstift in der Endstellung befindet, sich der Führungsstift nicht mehr in Längsrichtung zum distalen Ende der Nadel hin bewegen lässt, wodurch ein weiteres Herausbringen der Nadel aus der Sicherheitseinrichtung unterbunden wird.

Gemäß einer bevorzugten Ausführungsform umfasst daher das Stoppelement einen Scheitel, der auf einer Linie liegt, die senkrecht zu der Trennlinie angeordnet ist. Dies bedeutet, dass sich die Linie in eine Breitenrichtung des Spritzenkörpers erstreckt. Die Linie kann auch als Querlinie bezeichnet werden. Besonders vorteilhaft erstreckt sich ebenso das Stoppelement mit dem Scheitel in Richtung dieser Querlinie. Dies bedeutet, dass das Stoppelement im Wesentlichen in einem Winkel von 90°zu dem Tren nbereich angeordnet ist.

Durch die Anordnung eines solchen Stoppelements ist es also möglich, dass eine bereits benutze Spritze, also eine Spritze, dessen Stechelement bereits aus der Sicherheitseinrichtung herausgetreten ist, nicht noch einmal für einen Injektionsvorgang verwendet werden kann. Ebenso kann ein Patient oder ein Drittbenutzer sich vor und nach dem Injektionsvorgang nicht mehr an dem Stechelement verletzen, da das Stechelement jeweils von der Sicherheitseinrichtung umschlossen wird.

Gemäß einer bevorzugten Ausführungsform ist es daher auch denkbar, dass zusätzlich zu der bisher beschriebenen Sicherheitseinrichtung die Sicherheitseinrichtung ein Kappenelement und/oder einen Nadelschutz umfasst. Vorteilhaft ist das Kappenelement mit dem Nadelschutz ausgestattet. Weiter vorteilhaft ist das Kappenelement vor Gebrauch der Spritze von der Sicherheitseinrichtung abnehmbar, wodurch auch gegebenenfalls der Nadelschutz mit abgenommen werden kann. Somit kann für einen Benutzer der Spritze die Sicherheit der Sicherheitseinrichtung noch weiter erhöht werden, so dass die Gefahr einer Verletzung noch weiter minimiert werden kann.

Weitere vorteilhafte Ausführungsformen ergeben sich aus den Unteransprüchen.

Weitere Ziele, Vorteile und Zweckmäßigkeiten der vorliegenden Erfindung sind der nachfolgenden von der Beschreibung in Verbindung mit der Zeichnung zu entnehmen. Hierbei zeigen:
- Fig. 1: einen Ausschnitt des Spritzenkörpers;
- Fig. 2A: eine perspektivische Ansicht der Hülse;
- Fig. 2B: eine perspektivische Ansicht des Kappenelements;
- Fig. 3A: eine perspektivische Ansicht des Montageelements von oben;
- Fig. 3B: eine perspektivische Ansicht des Montageelement von unten;
- Fig. 4: einen Spritzenkörper mit darauf montierten Sicherheitseinrichtung in einem Längsschnitt;
- Fig. 5A: eine Spritze in der Ausgangsstellung;
- Fig. 5B: die Spritze gemäß Figur 5A im Moment der Überführung;
- Fig. 5C: die Spritze gemäß Figur 5A im Moment der Überführung mit einem Längsschnitt
- Fig. 5D: die Spritze gemäß Figur 5A in der Endstellung
- Fig. 5E: die Spritze gemäß Figur 5A in der Endstellung mit einem Längsschnitt
- Fig. 6: eine in ein Spritzennest eingehängte Spritze umfassend Spritzenkörper und Sicherheitseinrichtung

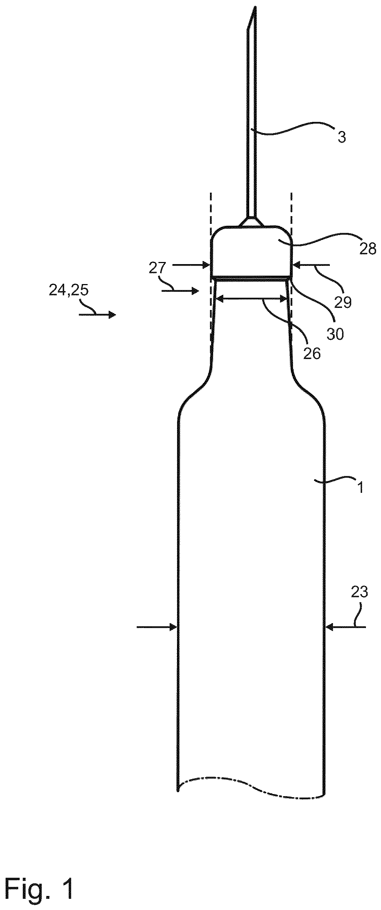

Figur 1 zeigt hierbei einen möglichen der Erfindung zugrunde liegenden Spritzenkörper 1. Handelsübliche Spritzenkörper 1 sind dabei im Wesentlichen zylinderförmig ausgestaltet, wie auch der vorliegende Spritzenkörper 1, und weist einen vorbestimmten Durchmesser 23 des auf. Vorliegend weist darüber hinaus der Spritzenkörper 1 an seinem distalen Ende 24 einen verengten Bereich 25 auf, der ebenfalls im Wesentlichen zylinderförmig ausgestaltet ist. Vorliegend weist jedoch der verengte Bereich 25 eine Kegelstumpfform auf, wobei der verengte Bereich in der Form eines Kegelstumpfes einen Durchmesser 26 an der Deckfläche (hier nicht gezeigt), also dem distalen Ende 27 des verengten Bereichs 25, auf, wobei der Durchmesser 26 des verengten Bereichs 25 kleiner ist als der Durchmesser 23 des Spritzenkörpers 1.

Weiter ist vorliegend an dem distalen Ende 27 des verengten Bereichs 25 eine Nadelhalterung 28 mit einem Stechelement 3, vorliegend einer Nadel 3, angeordnet, wobei diese Nadelhalterung 28 einen Durchmesser 29 aufweist, der größer ist als der Durchmesser 26 des verengten Bereichs 25, jedoch kleiner ist als der Durchmesser 23 des Spritzenkörpers 1. Weiter ist die Nadelhalterung 28 an dem distalen Ende 27 des verengten Bereichs 25 angeordnet, so dass ein Übergangsbereich 30 entsteht, wobei durch den Übergangsbereich 30 ein Bereich ausgebildet ist, an dem einerseits der Durchmesser 26 des verengten Bereichs 25 und der Durchmesser 29 der Nadelhalterung aufeinandertreffen und so der Durchmesser an sich sprunghaft verändert wird. Durch den Übergangsbereich 30 ist demnach eine Art Vorsprung ausgebildet.

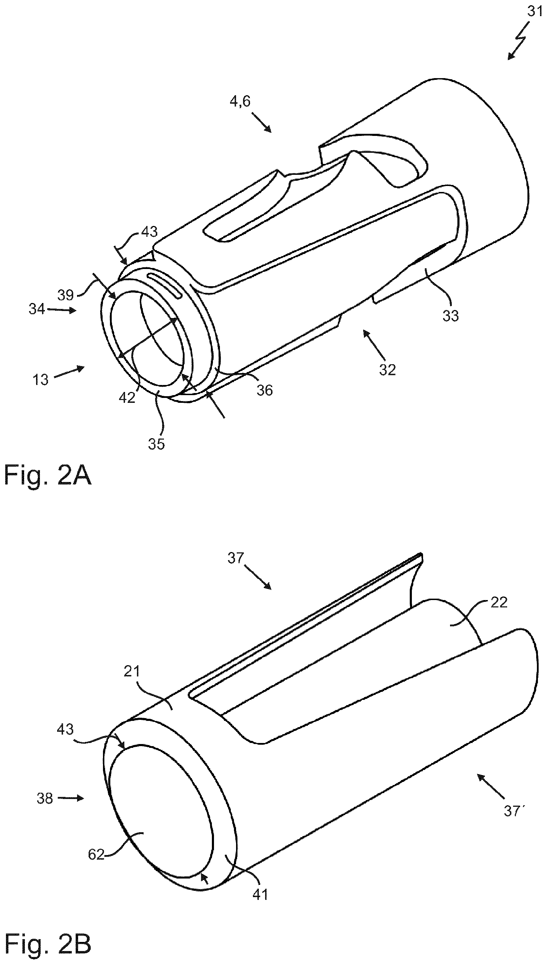

In der Figur 2A wird ein erster wichtiger Bestandteil der Sicherheitseinrichtung 2 in einer perspektivischen Ansicht gezeigt, nämlich die Hülse 31. Wie gut aus der Figur 2A zu erkennen ist, weist die Hülse eine Ausnehmung 4 auf, die die Führungskulisse 6 zur Führung des Führungsstiftes 5 (hier nicht gezeigt) ausbildet. Zusätzlich kann die Hülse einen Ausnahmebereich 32, der durch eine Berandung 33 begrenzt ist, aufweisen. Vorteilhaft ist der Ausnehmung 4 eine spiegelbildliche Ausnehmung 4' sowie dem Ausnahmebereich 32 ein spiegelbildlicher Ausnahmebereich 32' auf der Hülse 31 gegenüberliegend angeordnet.

Der Ausnahmebereich 32 dient vorliegend dazu, dass ein Kappenelement 21 (siehe Fig.2B) zur Sicherheitserhöhung der Sicherheitseinrichtung 2 geführt aufsteckbar ist. Durch die Berandung 33 des Ausnahmebereichs 32 ist das Kappenelement 21 mit der Hülse 31 zumindest in Wirkkontakt und bevorzugt zumindest kraftschlüssig verbunden.

An dem distalen Ende 34 der Hülse 31 weist die Hülse vorzugsweise eine Austrittsöffnung 13 auf, die vorliegend als ein Kreisring 35 ausgebildet ist, wobei an dem Kreisring 35 anschließend die Hülse 31 mit einem ringförmigen Bereich 36 fortgesetzt wird. Vorliegend weist der ringförmige Bereich 36 einen größeren Durchmesser als der Kreisring 35 auf. Besonders vorteilhaft sind der Kreisring 35 und der ringförmige Bereich 36 konzentrisch zueinander angeordnet. Vorteilhaft ist das Kappenelement 21 derart ausgestaltet, dass es auf den Kreisring 35 aufsetzbar ist, aber nicht auf dem ringförmigen Bereich 36, so dass bei einem Aufsetzen des Kappenelements 21 das Kappenelement 21 sowohl mit dem Kreisring 35 als auch mit dem ringförmigen Bereich 36 in Kontakt steht und die Aufsetzbewegung des Kappenelements 21 durch diese Ausgestaltung limitiert wird.

Der Figur 2B ist das Kappenelement 21, wie zuvor bereits angeschnitten, gezeigt. Vorliegend umfasst das Kappenelement 21 auch einen Nadelschutz 22 und weiter ein erstes Flügelelement 37 und ein zweites Flügelelement 37', die derart ausgestaltet sind, dass sie komplementär zu dem Ausnahmebereich 32 bzw. dem spiegelbildlichen Ausnahmebereich 32' ausgestaltet sind.

Der Nadelschutz 22 ist vorzugsweise im Wesentlichen zylindrisch ausgestaltet und ist vorteilhaft mit dem Kappenelement 21 fest verbindbar bzw. verbunden, wobei der Nadelschutz 22 vorzugsweise derart ausgestaltet ist, dass der Nadelschutz 22 in den Kreisring 35 hineinsteckbar ist. Dies bedeutet also, dass ein Außendurchmesser 40 (hier nicht gezeigt) des Nadelschutzes 22 höchstens dem Innendurchmesser 39 des Kreisrings 35 entspricht. Es ist jedoch vorstellbar, dass andere geometrische Formen anstelle eines Kreises verwendet werden.

Das distale Ende 38 des Kappenelements 21 ist vorliegend ebenfalls als ein Kreisring 41 ausgestaltet, dessen Innendurchmesser 43 mindestens dem Außendurchmesser 42 des Kreisrings 35 und höchstens dem Außendurchmesser 43 des ringförmigen Bereichs 36 entspricht. Dies bedeutet, dass der Kreisring 41 auf dem ringförmigen Bereich zu liegen kommt und somit miteinander wirkverbunden sind.

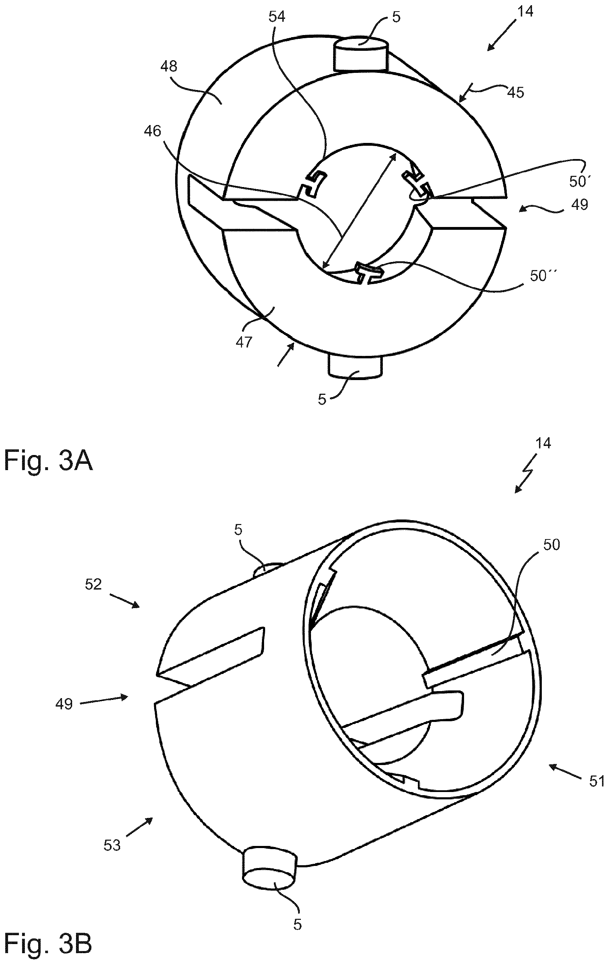

Die Figuren 3A und 3B zeigen das Montageelement 14, mittels welchem die Hülse 31 und somit die Sicherheitseinrichtung 2 im Ganzen mit dem Spritzenkörper 1 verbindbar ist, wobei die Figur 3A das Montageelement 14 in einer perspektivischen Ansicht von oben und die Figur 3B das Montageelement 14 in einer perspektivischen Ansicht von unten zeigt.

Das Montageelement 14 ist vorliegend im Wesentlichen eine zylindrische Form mit einem Außendurchmesser 45 und einem Innendurchmesser 46 auf, wobei durch das Bezugszeichen 47 das distale Ende des Montageelements 14 bezeichnet ist.

Vorliegend sind auf einer Mantelfläche 48 des Montageelements 14 zwei Führungsstifte 5 angeordnet, die sich bezüglich des Montageelements 14 gegenüber liegen. Diese Führungsstifte 5 sind dann in der Führungskulisse 6 der Hülse anordenbar und mit dieser wirkverbindbar.

Zusätzlich weist das Montageelement 14 eine oder mehrere, vorliegend zwei, Aussparungen 49 und ein oder mehrere, vorliegend drei, Rastelemente 50 auf, deren Funktionen nachstehend mit Bezug auf die Figur 3B dargestellt werden.

In der Figur 3B ist mit dem Bezugszeichen 51 ein proximales Ende 51 des Montageelements 14 bezeichnet. Wie zu erkennen ist, verlaufen die Rastelemente 50 des Montageelements 14 ansteigend vom proximalen Ende 51 zum distalen Ende 47 hin, d.h. die Rastelemente sind am distalen Ende 47 in radialer Richtung gesehen größer als am proximalen Ende 51.

Wird beim Zusammenfügen des Montageelements 14 mit dem Spritzenkörper 1, insbesondere mit dem verengten Bereich 25, das Montageelement 14 mit seinem proximalen Ende 51 zuerst auf die Nadelhalterung 28 geschoben, so wird durch den Dickenunterschied in radialer Richtung und ansteigend in axialer Richtung eine erste Hälfte 52 und eine zweite Hälfte 53 des Montageelements 14, die durch die Aussparungen 49 getrennt sind, voneinander wegbewegt. Es ist hierbei also erforderlich, dass das Montageelement 14 zumindest teilweise elastisch ausgebildet ist.

Passiert das distale Ende 47 des Montageelements 14 den Übergangsbereich 30, so bewegen sich die erste 52 und die zweite Hälfte 53 durch die elastische Ausgestaltung aufeinander zu, so dass schlussendlich das Montageelement 14 und somit die Sicherheitseinrichtung 2 auf dem Spritzenkörper 1 aufgeclipst ist.

Zur Sicherung dieser Clipverbindung weisen die Rastelemente 50 an ihrem distalen Ende 50' Sicherungsabschnitte 50" auf, die sich in Umfangsrichtung eines Innenkreises 54 des Montageelements 14 erstrecken und mit dem Übergangsbereich 30 in mechanischen Wirkkontakt stehen.

Besonders bevorzugt ist die Sicherheitseinrichtung 2 umfassend die Hülse 31, das Montageelement 14 und das Kappenelement 21 bereits vormontierbar und als Ganzes mit dem Spritzenkörper 1 mittels des Montageelements 14 verbindbar.

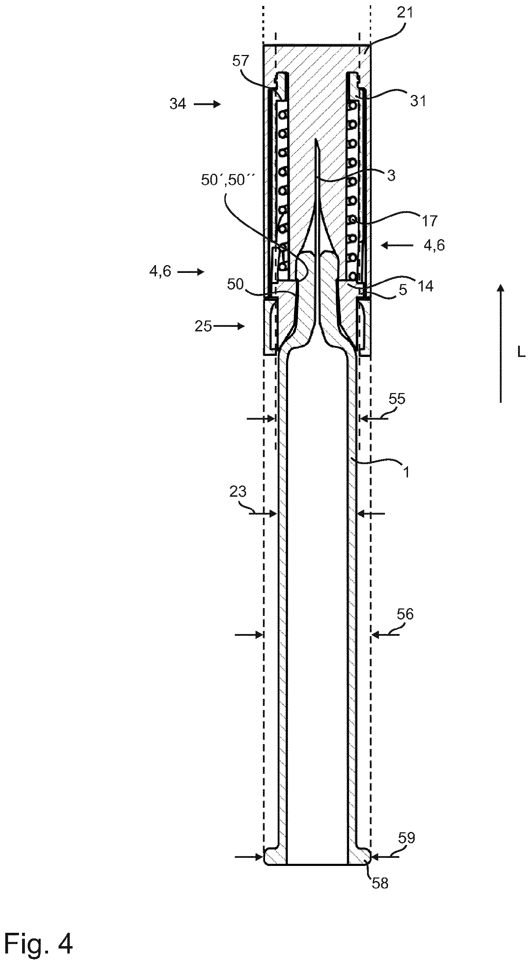

Die Figur 4 zeigt einen Spritzenkörper 1 mit einer darauf montierten Sicherheitseinrichtung 2 in einem Längsschnitt.

Die Sicherheitseinrichtung 2 umfasst dabei zusätzlich zu dem Kappenelement 21, der Hülse 31 und dem Montageelement 14 ein Federelement 17, das vorliegend als eine Spiralfeder 17 ausgebildet ist.

Wie deutlich zu erkennen ist, stehen die Führungsstifte 5 mit der Ausnehmung 4 bzw. der Führungskulisse 6 in Kontakt, so dass die Führungsstifte durch die Führungskulisse bei einer Relativbewegung des Spritzenkörpers gegenüber der Sicherheitseinrichtung geführt werden.

Das Montageelement 14 ist mit dem verengten Bereich 25 mittels der Rastelemente 50 und deren Sicherungsabschnitte 50" miteinander verclipst und mittels der Führungsstifte 5 mit der Hülse 31 verbunden.

Die Hülse 31 umfasst weiter an seinem distalen Ende 34 einen innenliegenden Auflagebereich 57, der mit dem Federelement 17 wirkverbindbar ist. Das Federelement 17 wird also einerseits durch den Auflagebereich 57 und andererseits durch das Montageelement 14 in der Hülse 31 gehalten und ist so gegen ein Herausfallen gesichert.

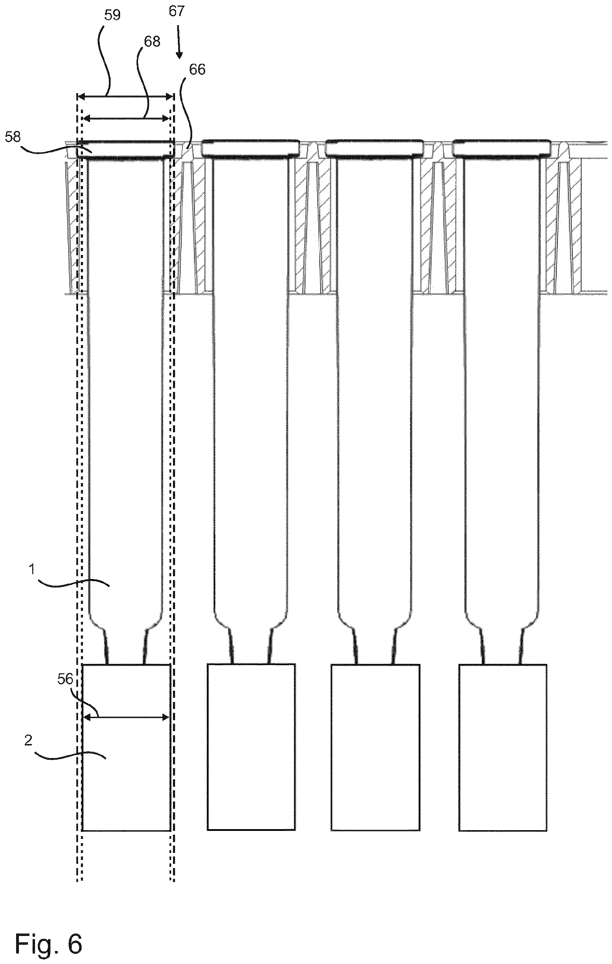

Die Dimensionen der Sicherheitseinrichtung 2 sind derart gewählt, dass die Hülse 31 einen Innendurchmesser 55 aufweist, der größer ist als der Durchmesser 23 des Spritzenkörpers 1, so dass der Spritzenkörper 1 bei einer Bewegung in Längsrichtung L nach vorne, wobei die Bewegungsrichtung gekennzeichnet ist durch einen Pfeil L, gegenüber der Sicherheitseinrichtung 2 der Spritzenkörper in die Hülse 31 hineinbewegbar ist. Gleichzeitig ist der Außendurchmesser 56 der Hülse 31 bzw. der Sicherheitseinrichtung 2 so gewählt, dass er höchstens einem maximalen Durchmesser einer am proximalen Ende des Spritzenkörpers 1 angebrachten Haltevorrichtung 58 zum Halten und sicheren Setzen der Spritze entspricht. Der Zweck dieser Größeneinschränkung wir mit Bezug auf Figur 6 genauer dargestellt.

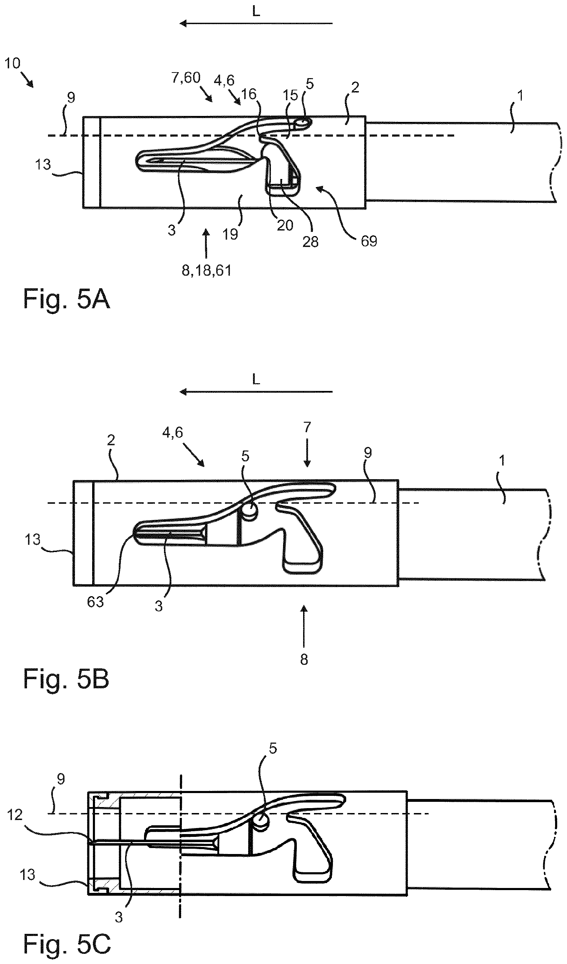

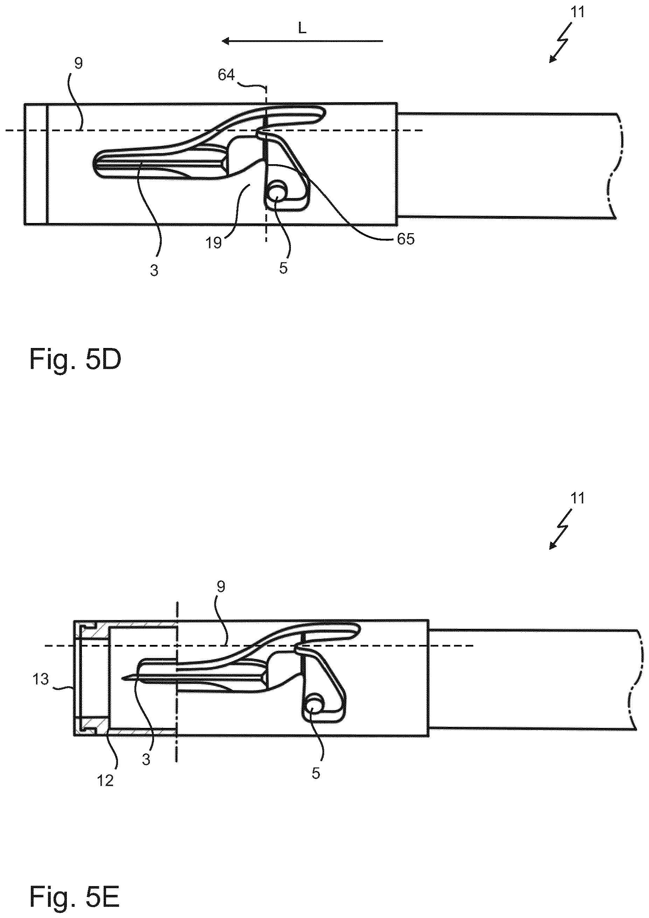

Mit Bezug auf die Figuren 5A-5E wird die Sicherheitseinrichtung 2 noch einmal im Detail vorgestellt, insbesondere die Bewegung des Führungsstiftes 5 und des Spritzenkörpers 1 relativ zur Sicherheitseinrichtung 2 sowie die Position der Nadel 3. Die Sicherheitseinrichtung 2 ist in den Figuren 5A-5E zur Übersichtlichkeit ohne Kappenelement 21 und Nadelschutz 22 dargestellt.

In der Figur 5A ist die Anordnung bestehend aus Spritzenkörper 1 und Sicherheitseinrichtung 2 in einer Ausgangsstellung 10 zu erkennen. Unter einer Ausgangsstellung 10 wird eine noch nicht benutze Spritze verstanden.

Der Führungsstift 5 befindet sich ebenso in einer Ausgangsstellung 10 und ist in dem ersten Kulissenbereich 7 angeordnet. Der erste Kulissenbereich 7 wird von dem zweiten Kulissenbereich 8 durch die fiktive Trennlinie 9 und dem Trennbereich 15, der einen Scheitelpunkt 16 aufweist, getrennt. Der Scheitelpunkt 16 liegt hierbei auf der fiktiven Trennlinie 9. Der erste Kulissenbereich 7 umfasst einen Kulissenabschnitt 60, der zweite Kulissenbereich 8 einen zweiten Kulissenabschnitt 61. Ferner umfasst der zweiten Kulissenbereich 8 ein Stoppelement 19.

Der erste Kulissenbereich 7 ist hierbei im Wesentlichen I-förmig ausgebildet und umfasst einen kurvenförmigen ersten Kurvenabschnitt 60, wobei der zweite Kulissenbereich 8 im Wesentlichen eine L-förmige Gestalt aufweist mit einem zweiten kurvenförmigen Kurvenabschnitt 61, wobei der zweite Kurvenabschnitt 61 aus mehreren Teilen besteht.

Vorzugsweise wird das distale Ende 34 der Hülse direkt auf die Haut aufgesetzt, so dass die Austrittsöffnung 13 mit der Haut in Kontakt steht. Wird der Spritzenkörper 1 nun relativ zu der Sicherheitseinrichtung 2 in Längsrichtung L bewegt, so wird der Führungsstift 5 durch den ersten Kulissenabschnitt 60 geführt, wodurch das Montageelement 14 um den verengten Bereich 25 herum bewegt wird. Das Montageelement 14 ist also in axialer Richtung fest montiert, kann sich aber in radialer Richtung um den verengten Bereich 25 herum frei drehen.

Überschreitet der Führungsstift 5 die fiktive Trennlinie 9, so wird der Führungsstift 5 von dem ersten Kulissenbereich 7 in den zweiten Kulissenbereich 8 überführt, wie in der Figur 5B bzw. 5C gut zu erkennen ist. Im Moment der Überführung ist dabei das distale Ende 12 des Stechelements 3 auf Höhe der Austrittsöffnung 13 angeordnet. Das Stechelement 3 ist also gerade im Begriff, aus der Sicherheitseinrichtung 2 herauszutreten, so dass eine Injektion möglich ist.

Das Stechelement 3 kann dann weiter aus der Sicherheitseinrichtung 2 herausbewegt werden, solange bis der Führungsstift 5 ein distales Ende 63 des zweiten Kulissenbereichs 8 erreicht hat und dadurch eine weitere Bewegung in Längsrichtung verhindert wird.

Wurde die Injektion durchgeführt bzw. das Stechelement 3 aus der Sicherheitseinrichtung 2 herausbewegt, so vermindert der Benutzer den Druck auf die Spritze, wodurch durch die Federkraft des Federelements 17 der Spritzenköper 1 gegenüber der Sicherheitseinrichtung 2 entgegen der Längsrichtung L bewegt wird. Das Stechelement wird also durch das Federelement 17 automatisch in die Sicherheitseinrichtung 2 zurückbewegt. Der Führungsstift 5 bewegt sich hierbei, je nachdem wie weit das Stechelement 3 herausbewegt wurde, entlang des zweiten Kulissenabschnitts 61 und/oder auf einer Linie (hier nicht gezeigt), die parallel zur Längsrichtung verläuft.

Ab einer gewissen Position des Führungsstifts 5 im zweiten Kulissenbereich 8 ist der Führungsstift 5 mit dem zweiten Kulissenabschnitt 61 in Kontakt und wird von diesem geführt. Der zweite Kulissenabschnitt ist derart ausgestaltet, dass er, mit Unterstützung der Rückführbewegung durch das Federelement 17, den Führungsstift automatisch in eine Endstellung 11 überführt und steht in Kontakt mit einem Stoppelement 19 in Kontakt. Das Stoppelement 19 umfasst vorzugsweise einen Scheitel 20, der auf der Querlinie 64, die senkrecht zur Trennlinie 9 verläuft, angeordnet ist. Der Scheitel 20 dient zur besseren Führung des Führungsstifts 5 innerhalb des zweiten Kulissenbereichs 8. Vorliegend umfasst das Stoppelement weiter einen Abschnitt 65, an dem der Führungsstift 5 anliegt. Vorliegend liegt dieser Abschnitt 65 ebenso auf der Querlinie 64, wobei jedoch auch andere Ausgestaltungen des Abschnitts 65 denkbar sind. Beispielsweise kann der Abschnitt 65 auch eine Kurvenbahn aufweisen.

Dabei ist die Form des Abschnitts 65 immer derart zu wählen, dass bei einem Versuch, die Spritze erneut zu betätigen, der Führungsstift 5 in seiner Endstellung 11 verbleibt.

In der Figur 6 wird Bezug genommen auf die Figur 4, in der beschrieben worden ist, welche Dimension die Sicherheitseinrichtung 2 vorteilhaft haben sollte. Zu sehen ist weiterhin ein Ausschnitt eines Spritzennests 66, wobei die Spritzen jeweils in eine Öffnung 67 des Spritzennests 66 eingehängt sind. Um eine Spritze in das Spritzennest 66 einhängen zu können, ist der Durchmesser 59 der Haltevorrichtung größer gewählt als der Durchmesser 68 einer Öffnung 67. Um den Spritzenkörper 1 samt Sicherheitseinrichtung 2 in eine Öffnung 67 einhängen zu können, muss also der Außendurchmesser 56 der Hülse 31 bzw. der Außendurchmesser 56 der Sicherheitseinrichtung 2 kleiner gewählt sein als der Durchmesser 59 der Haltevorrichtung 58. Es ist daher möglich, die Spritze bereits mit der Sicherheitseinrichtung 2 in das Spritzennest einzuhängen, zu befüllen und zu sterilisieren.

### Bezugszeichenliste

- 1: Spritzenkörper
- 2: Sicherheitseinrichtung
- 3: Stechelement
- 4: Ausnehmung
- 4': spiegelbildliche Ausnehmung
- 5: Führungsstift
- 6: Führungskulisse
- 7: erster Kulissenbereich
- 8: zweiter Kulissenbereich
- 9: fiktive Trennlinie
- 10: Ausgangstellung
- 11: Endstellung
- 12: distales Ende des Stechelements
- 13: Austrittsöffnung
- 14: Montageelement
- 15: Trennbereich
- 16: Scheitelpunkt des Trennbereichs
- 17: Federelement
- 18: Kulisse des zweiten Kulissenbereichs
- 19: Stoppelement
- 20: Scheitel des Stoppelements
- 21: Kappenelement
- 22: Nadelschutz
- 23: Durchmesser des Spritzenkörpers
- 24: distales Ende des Spritzenkörpers
- 25: verengter Bereich des Spritzenkörpers
- 26: Durchmesser des verengten Bereichs
- 27: distales Ende des verengten Bereichs
- 28: Nadelhalterung
- 29: Durchmesser Nadelhalterung
- 30: Übergangsbereich
- 31: Hülse
- 32: Ausnahmebereich
- 32': spiegelbildlicher Ausnahmebereich
- 33: Berandung
- 34: distales Ende der Hülse
- 35: Kreisring
- 36: ringförmiger Bereich
- 37: erstes Flügelelement
- 37': zweites Flügelelement
- 38: distales Ende des Kappenelements
- 39: Innendurchmesser des Kreisrings
- 40: Außendurchmesser Nadelschutz
- 41: Kreisring
- 42: Außendurchmesser Kreisring
- 43: Innendurchmesser Kreisring
- 44: Außendurchmesser ringförmiger Bereich
- 45: Außendurchmesser des Montageelements
- 46: Innendurchmesser des Montageelements
- 47: distales Ende des Montageelements
- 48: Mantelfläche des Montageelements
- 49: Aussparung
- 50: Rastelement
- 50': distales Ende des Rastelements
- 50": Sicherungsabschnitt
- 51: proximales Ende des Montageelements
- 52: erste Hälfte des Montageelements
- 53: zweite Hälfte des Montageelements
- 54: Innenkreis
- 55: Innendurchmesser der Hülse
- 56: Außendurchmesser der Hülse
- 57: Auflagebereich
- 58: Haltevorrichtung
- 59: Durchmesser der Haltevorrichtung
- 60: erster Kulissenabschnitt
- 61: zweiter Kulissenabschnitt
- 62: distales Ende des Kappenelements
- 63: distales Ende der Führungskulisse
- 64: Querlinie
- 65: Abschnitt des Stoppelements
- 66: Spritzennest
- 67: Öffnung
- 68: Durchmesser der Öffnung

## Patentansprüche

1. Spritzenkörper (1) mit einer endseitig angeordneten Sicherheitseinrichtung (2) zur Vermeidung von Stichverletzungen, wobei der Spritzenkörper (1) ein endseitig angeordnetes Stechelement (3) mit einem distalen Ende (12) umfasst und die Sicherheitseinrichtung (2) eine Ausnehmung (4) und mindestens einen Führungsstift (5) aufweist, mittels welcher eine Führungskulisse (6) ausgebildet ist, um den Führungsstift (5) bei einer Relativbewegung des Spritzenkörpers (1) zur Sicherheitseinrichtung (2) in einer Längsrichtung (L) des Spritzenkörpers (1) zu führen, wobei in einer Ausgangsstellung (10), in der das distale Ende (12) des Stechelement (3) innerhalb der Sicherheitseinrichtung (2) angeordnet ist, der Führungsstift (5) in einem ersten Kulissenbereich (7) der Führungskulisse (6) angeordnet ist und in einer Endstellung (11), in der das distale Ende (12) des Stechelement (3) durch eine Austrittsöffnung (13) aus der Sicherheitseinrichtung (2) herausbewegt ist, der Führungsstift (5) in einem zweiten Kulissenbereich (8) der Führungskulisse (6) angeordnet ist, wobei zwischen dem ersten (7) und dem zweiten Kulissenbereich (8) zumindest teilweise ein Trennbereich (15) mit einem Scheitelpunkt (16) angeordnet ist, wobei in Längsrichtung (L) durch den Scheitelpunkt (16) eine fiktive Trennlinie (9), welche den ersten (7) und den zweiten Kulissenbereich (8) trennt, verläuft,
**dadurch gekennzeichnet, dass**
der erste Kulissenbereich (7) einen ersten kurvenförmigen Abschnitt (60) aufweist, an dem der Führungsstift (5) durch die Relativbewegung des Spritzenkörpers (1) bis zu der fiktiven Trennlinie (9) geführt ist, wobei der zweite Kulissenbereich (8) einen zweiten kurvenförmigen Abschnitt (61) aufweist, an dem der Führungsstift (5) durch die Relativbewegung des Spritzenkörpers (1) nach Überschreiten der fiktiven Trennlinie (9) geführt ist, wobei bei Überschreiten der fiktiven Trennlinie (9) das distale Ende (12) des Stechelements (3) auf der Höhe der Austrittsöffnung (13) der Sicherheitseinrichtung (2) angeordnet ist.

2. Spritzenkörper (1) nach Anspruch 1,
**dadurch gekennzeichnet, dass**
der Führungsstift (5) auf einem Montageelement (14) der Sicherheitseinrichtung (2) angeordnet ist.

3. Spritzenkörper (1) nach Anspruch 2,
**dadurch gekennzeichnet, dass**
der Spritzenkörper (1) und die Sicherheitseinrichtung (2) mittels des Montageelements (14) miteinander verbindbar sind.

4. Spritzenkörper (1) nach einem der vorhergehenden Ansprüche,
**dadurch gekennzeichnet, dass**
die Sicherheitseinrichtung (2) mindestens ein Federelement (17) aufweist, das mit dem Spritzenkörper (1) wirkverbunden ist und der Relativbewegung des Spritzenkörpers (1) zu der Sicherheitseinrichtung (2) entgegenwirkt.

5. Spritzenkörper (1) nach einem der vorhergehenden Ansprüche,
**dadurch gekennzeichnet, dass**
wenn der Führungsstift (5) sich in dem zweiten Kulissenbereich (8) befindet, der Führungsstift (5) mittels einer Kulisse (18, 61) des zweiten Kulissenbereichs (8) in einen Endbereich (69) überführt wird und der Führungsstift (5) mit einem Stoppelement (19) wirkverbunden ist.

6. Spritzenkörper (1) nach Anspruch 5,
**dadurch gekennzeichnet, dass**
das Stoppelement (19) einen Scheitel (20) umfasst, der auf einer Linie (64) liegt, die senkrecht zu der Trennlinie (9) angeordnet ist.

7. Spritzenkörper (1) nach einem der vorhergehenden Ansprüche,
**dadurch gekennzeichnet, dass**
die Sicherheitseinrichtung (2) ein Kappenelement (21) und einen Nadelschutz (22) umfasst.

## Claims

1. Syringe body (1) comprising a safety device (2) arranged at one end for preventing pricking injuries, the syringe body (1) comprising a piercing element (3) that is arranged at one end and has a distal end (12), and the safety device (2) having a recess (4) and at least one guide pin (5), by means of which recess a guide slot (6) is formed in order to guide the guide pin (5), in a movement of the syringe body (1) relative to the safety device (2), in a longitudinal direction (L) of the syringe body (1), the guide pin (5) being arranged in a first slot region (7) of the guide slot (6) in an initial position (10), in which the distal end (12) of the piercing element (3) is arranged inside the safety device (2), and the guide pin (5) being arranged in a second slot region (8) of the guide slot (6) in an end position (11), in which the distal end (12) of the piercing element (3) is moved out of the safety device (2) through an exit opening (13), a separating region (15) having an apex (16) being arranged at least in part between the first (7) and the second slot region (8), a notional separation line (9) which separates the first (7) and the second slot region (8) extending in the longitudinal direction (L) through the apex point (16),
**characterised in that**
the first slot region (7) has a first curved portion (60) on which the guide pin (5) is guided by the relative movement of the syringe body (1) as far as the notional separation line (9), the second slot region (8) having a second curved portion (61) on which the guide pin (5) is guided by the relative movement of the syringe body (1) after crossing the notional separation line (9), the distal end (12) of the piercing element (3) being arranged at the level of the exit opening (13) of the safety device (2) when the notional separation line (9) is crossed.

2. Syringe body (1) according to claim 1,
**characterised in that**
the guide pin (5) is arranged on a mounting element (14) of the safety device (2).

3. Syringe body (1) according to claim 2,
**characterised in that**
the syringe body (1) and the safety device (2) can be interconnected by means of the mounting element (14).

4. Syringe body (1) according to any of the preceding claims,
**characterised in that**
the safety device (2) comprises at least one spring element (17) which is operatively connected to the syringe body (1) and counteracts the movement of the syringe body (1) relative to the safety device (2).

5. Syringe body (1) according to any of the preceding claims,
**characterised in that**
when the guide pin (5) is located in the second slot region (8), the guide pin (5) is moved into an end region (69) by means of a slot (18, 61) of the second slot region (8), and the guide pin (5) is operatively connected to a stop element (19).

6. Syringe body (1) according to claim 5,
**characterised in that**
the stop element (19) comprises an apex (20) that is positioned on a line (64) which is arranged perpendicularly to the separation line (9).

7. Syringe body (1) according to any of the preceding claims,
**characterised in that**
the safety device (2) comprises a cap element (21) and a needle guard (22).

## Revendications

1. Seringue (1) comportant un dispositif de sécurité (2) disposé côté extrémité pour éviter des blessures par piqûre, la seringue (1) comportant un élément de percement (3) disposé côté extrémité avec une extrémité distale (12) et le dispositif de sécurité (2) présentant une cavité (4) et au moins une broche de guidage (5), au moyen de laquelle est formée une coulisse de guidage (6) pour guider la broche de guidage (5) lors d'un mouvement relatif de la seringue (1) par rapport au dispositif de sécurité (2) dans une direction longitudinale (L) de la seringue (1), où, dans une position de départ (10), dans laquelle l'extrémité distale (12) de l'élément de percement (3) est disposée à l'intérieur du dispositif de sécurité (2), la broche de guidage (5) est disposée dans une première zone de coulisse (7) de la coulisse de guidage (6) et, dans une position finale (11), dans laquelle l'extrémité distale (12) de l'élément de percement (3) est sortie du dispositif de sécurité (2) par une ouverture de sortie (13), la broche de guidage (5) est disposée dans une deuxième zone de coulisse (8) de la coulisse de guidage (6), où entre la première (7) et la deuxième zone de coulisse (8) est disposée au moins en partie une zone de séparation (15) avec un sommet (16), où dans la direction longitudinale (L) à travers le sommet (16) s'étend une ligne de séparation fictive (9), laquelle sépare la première (7) et la deuxième zone de coulisse (8),
**caractérisé par le fait que**
la première zone de coulisse (7) présente une première section courbe (60), sur laquelle la broche de guidage (5) est guidée par le mouvement relatif de la seringue (1) jusqu'à la ligne de séparation fictive (9), la deuxième zone de coulisse (8) présentant une deuxième section courbe (61), sur laquelle la broche de guidage (5) est guidée par le mouvement relatif de la seringue (1) après dépassement de la ligne de séparation fictive (9), l'extrémité distale (12) de l'élément de percement (3) étant disposée au niveau de l'ouverture de sortie (13) du dispositif de sécurité (2) lors du dépassement de la ligne de séparation fictive (9).

2. Seringue (1) selon la revendication 1,
**caractérisé par le fait que**
la broche de guidage (5) est disposée sur un élément de montage (14) du dispositif de sécurité (2).

3. Seringue (1) selon la revendication 2,
**caractérisé par le fait que**
la seringue (1) et le dispositif de sécurité (2) sont aptes à être reliés l'un à l'autre au moyen de l'élément de montage (14).

4. Seringue (1) selon l'une des revendications précédentes,
**caractérisé par le fait que**
le dispositif de sécurité (2) présente au moins un élément ressort (17), qui est relié fonctionnellement avec la seringue (1) et s'oppose au mouvement de la seringue (1) par rapport au dispositif de sécurité (2).

5. Seringue (1) selon l'une des revendications précédentes,
**caractérisé par le fait que**
lorsque la broche de guidage (5) se trouve dans la deuxième zone de coulisse (8), la broche de guidage (5) est transférée au moyen d'une coulisse (18, 61) de la deuxième zone de coulisse (8) dans une zone d'extrémité (69) et la broche de guidage (5) est reliée fonctionnellement avec un élément de butée (19).

6. Seringue (1) selon la revendication 5,
**caractérisé par le fait que**
l'élément de butée (19) comporte un sommet (20) qui se trouve sur une ligne (64) qui est disposée perpendiculairement à la ligne de séparation (9).

7. Seringue (1) selon l'une des revendications précédentes,
**caractérisé par le fait que**
le dispositif de sécurité (2) comporte un élément formant capuchon (21) et un protecteur d'aiguille (22).
